(19) Europäisches Patentamt
European Patent Office
Office européen des brevets

(11) Veröffentlichungsnummer: **0 025 867**
**B1**

# (12) EUROPÄISCHE PATENTSCHRIFT

(45) Veröffentlichungstag der Patentschrift: **28.09.83**

(51) Int. Cl.³: **C 07 D 498/10,** C 08 K 5/35 // (C07D498/10, 263/00, 221/00)

(21) Anmeldenummer: **80104875.2**

(22) Anmeldetag: **16.08.80**

(54) **Alkylierte Diazaspirodecane, ihre Herstellung und Verwendung als Lichtschutzmittel.**

(30) Priorität: **21.08.79 DE 2933732**

(43) Veröffentlichungstag der Anmeldung: **01.04.81 Patentblatt 81/13**

(45) Bekanntmachung des Hinweises auf die Patenterteilung: **28.09.83 Patentblatt 83/39**

(84) Benannte Vertragsstaaten: **BE CH DE FR GB IT LI NL**

(56) Entgegenhaltungen:
**EP-A-0 001 207**
**EP-A-0 008 084**
**EP-A-0 017 617**
**DE-A-2 227 689**
**DE-A-2 606 026**
**DE-A-2 634 957**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Wiezer, Hartmut, Dr., Hans-Fischer-Strasse 6, D-8906 Gersthofen (DE)**
Erfinder: **Pfahler, Gerhard, Dr., Karlsbader Strasse 27, D-8900 Augsburg (DE)**
Erfinder: **Häberlein, Harald, Dr., Schlesierstrasse 2, D-8906 Gersthofen (DE)**
Erfinder: **Nowy, Günther, Dr., Hans-Fischer-Strasse 4, D-8906 Gersthofen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 025 867 B1

BUNDESDRUCKEREI BERLIN

Alkylierte Diazaspirodecane, ihre Herstellung und Verwendung als Lichtschutzmittel

Die Erfindung betrifft neue Polyalkyldiazaspirodecane, ein Verfahren zu ihrer Herstellung sowie ihre Verwendung zum Stabilisieren von organischen Polymeren gegen den licht- und wärmeinduzierten Abbau.

Die neuen Polyalkyldiazaspirodecane entsprechen der allgemeinen Formel (I)

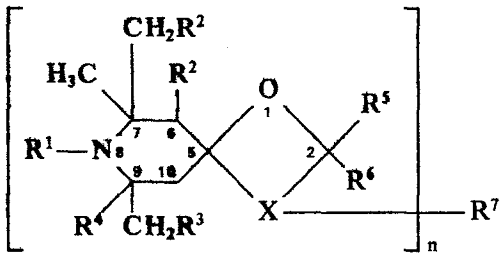

(I)

in welcher

X die Bedeutung einer Gruppe der Formel (II) oder (III) hat,

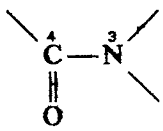 (II)

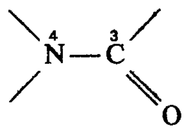 (III)

wobei die Indices 3 bzw. 4 die Ringposition im Diazaspirodecansystem angeben und die freie Valenz des Stickstoffatoms die Verknüpfung mit $R^7$ bewirkt,

$R^1$ Wasserstoff, Sauerstoff oder $C_1$- bis $C_{12}$-Alkyl, vorzugsweise Wasserstoff, Sauerstoff oder $C_1$- bis $C_4$-Alkyl und insbesondere Wasserstoff ist,

$R^2$ und $R^3$ entweder gleich sind und Wasserstoff oder eine $C_1$- bis $C_5$-Alkylgruppe, vorzugsweise Wasserstoff oder eine Methylgruppe und insbesondere Wasserstoff bedeuten, wobei dann

$R^4$ eine Methylgruppe ist oder auch

$R^2$ Wasserstoff oder $C_1$- bis $C_5$-Alkyl ist und

$R^3$ und $R^4$ zusammen mit den Kohlenstoffatomen, an die sie gebunden sind, eine $C_5$- oder $C_6$-Cycloalkylgruppe oder eine Gruppe der Formel

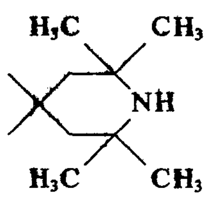

darstellen,

$R^5$ für Wasserstoff, $C_1$- bis $C_{30}$-Alkyl, vorzugsweise $C_1$- bis $C_{18}$-Alkyl, insbesondere $C_1$- bis $C_5$-Alkyl, für eine unsubstituierte oder durch Chlor oder $C_1$- bis $C_4$-Alkyl-substituierte Phenyl- oder Naphthylgruppe, vorzugsweise die erstgenannte, oder für eine unsubstituierte oder durch $C_1$- bis $C_4$-Alkyl substituierte $C_7$- bis $C_{12}$-Phenylalkylgruppe, vorzugsweise für eine Benzylgruppe, steht,

$R^6$ Wasserstoff, $C_1$- bis $C_{30}$-Alkyl, vorzugsweise $C_1$- bis $C_{18}$-Alkyl, insbesondere $C_1$- bis $C_{13}$-Alkyl, eine unsubstituierte oder durch Chlor oder $C_1$- bis $C_4$-Alkyl substituierte Phenyl- oder Naphthylgruppe, vorzugsweise eine Phenylgruppe, eine unsubstituierte oder durch $C_1$- bis $C_4$-Alkyl substituierte $C_7$- bis $C_{12}$-Phenylalkylgruppe, vorzugsweise eine Benzylgruppe ist, oder

$R^5$ und $R^6$ zusammen mit dem sie bindenden Kohlenstoffatom die Bedeutung einer unsubstituierten oder durch bis zu vier $C_1$- bis $C_4$-Alkylgruppen, vorzugsweise Methylgruppen substituierten $C_5$- bis $C_{18}$-, vorzugsweise $C_5$- bis $C_{12}$-Cycloalkylgruppe oder einer Gruppe der Formel

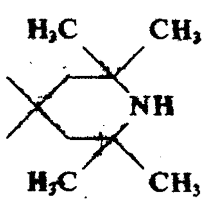

haben;

n bedeutet 1 oder 2 und

$R^7$ ist bei n = 1 eine Alkylgruppe mit 1 bis 30, vorzugsweise 1 bis 18 und insbesondere 1 bis 6 Kohlenstoffatomen, eine Alkenylgruppe mit 2 bis 18, vorzugsweise 3 bis 12 und insbesondere 3 bis 6 Kohlenstoffatomen, eine gegebenenfalls durch eine $C_1$- bis $C_4$-Alkylgruppe substituierte Phenylalkylgruppe mit 7 bis 18, vorzugsweise 7 bis 10 und insbesondere 7 Kohlenstoffatomen oder eine Cycloalkylgruppe mit 5 bis 12 Kohlenstoffatomen und
bei n = 2 eine Alkylengruppe mit 2 bis 30, vorzugsweise 2 bis 18 und insbesondere 4 bis 12 Kohlenstoffatomen, eine Alkenylgruppe mit 2 bis 30, vorzugsweise 2 bis 18 und insbesondere 2 bis 6 Kohlenstoffatomen oder eine Phenyldialkylengruppe mit 8 bis 18, vorzugsweise 8 bis 10 und insbesondere 8 Kohlenstoffatomen.

Eingeschlossen sind ferner die Salze mit nichtoxidierenden Mineralsäuren, aliphatischen Sulfon- oder Phosphonsäuren mit 1 bis 30, vorzugsweise 1 bis 18 C-Atomen, gegebenenfalls alkylierten aromatischen Sulfon- oder Phosphonsäuren mit 6 bis 25, vorzugsweise 6 bis 18 C-Atomen, wobei 1 bis 3 Alkylgruppen mit 1 bis 16 C-Atomen vorhanden sein können, ferner aliphatischen Mono- oder Dicarbonsäuren mit 2 bis 34, vorzugsweise 2 bis 18 C-Atomen oder aliphatischen Polycarbonsäuren mit bis zu 4 Carboxylgruppen und insgesamt 16 C-Atomen oder auch aromatischen Carbonsäuren mit bis zu 4 Carboxylgruppen und bis zu 25 C-Atomen, vorzugsweise bis zu 2 Carboxylgruppen und bis zu 19 C-Atomen.

Beispiele für erfindungsgemäße Polyalkyldiazaspirodecane sind:

(1) 2,2,3,7,7,9,9-Heptamethyl-1-oxa-3,8-diaza-4-oxo-spiro-(4,5)-decan
(2) 2,2,7,7,9,9-Hexamethyl-3-ethyl-1-oxa-3,8-diaza-4-oxo-spiro-(4,5)-decan
(3) 2,2,7,7,9,9-Hexamethyl-3-propyl-1-oxa-3,8-diaza-4-oxo-spiro-(4,5)-decan
(4) 2,2,7,7,9,9-Hexamethyl-3-butyl-1-oxa-3,8-diaza-4-oxo-spiro-(4,5)-decan
(5) 2,2,7,7,9,9-Hexamethyl-3-pentyl-1-oxa-3,8-diaza-4-oxo-spiro-(4,5)-decan
(6) 2,2,7,7,9,9-Hexamethyl-3-hexyl-1-oxa-3,8-diaza-4-oxo-spiro-(4,5)-decan
(7) 2,2,7,7,9,9-Hexamethyl-3-nonyl-1-oxa-3,8-diaza-4-oxo-spiro-(4,5)-decan
(8) 2,2,7,7,9,9-Hexamethyl-3-dodecyl-1-oxa-3,8-diaza-4-oxo-spiro-(4,5)-decan
(9) 2,2,7,7,9,9-Hexamethyl-3-octadecyl-1-oxa-3,8-diaza-4-oxo-spiro-(4,5)-decan
(10) 2,2,7,7,9,9-Hexamethyl-3-benzyl-1-oxa-3,8-diaza-4-oxo-spiro-(4,5)-decan
(11) 1',4'-Bis-[2,2,7,7,9,9-hexamethyl-1-oxa-3,8-diaza-4-oxo-spiro-(4,5)-decyl-3-]-butan
(12) 1',6'-Bis-[2,2,7,7,9,9-hexamethyl-1-oxa-3,8-diaza-4-oxo-spiro-(4,5)-decyl-3-]-hexan
(13) 1',10'-Bis-[2,2,7,7,9,9-hexamethyl-1-oxa-3,8-diaza-4-oxo-spiro-(4,5)-decyl-3-]-decan
(14) 2,2,4,7,7,9,9-Heptamethyl-1-oxa-3-oxo-4,8-diaza-spiro-(4,5)-decan
(15) 2,2,7,7,8,9,9-Heptamethyl-4-butyl-1-oxa-3-oxo-4,8-diaza-spiro-(4,5)-decan
(16) 2,2,7,7,9,9-Hexamethyl-4-ethyl-1-oxa-3-oxo-4,8-diaza-spiro-(4,5)-decan
(17) 2,2,7,7,9,9-Hexamethyl-4-butyl-1-oxa-3-oxo-4,8-diaza-spiro-(4,5)-decan
(18) 2,2,7,7,9,9-Hexamethyl-4-hexyl-1-oxa-3-oxo-4,8-diaza-(4,5)-decan
(19) 2,2,7,7,9,9-Hexamethyl-4-dodecyl-1-oxa-3-oxo-4,8-diaza-spiro-(4,5)-decan
(20) 2,2,7,7,9,9-Hexamethyl-4-octadecyl-1-oxa-3-oxo-4,8-diaza-spiro-(4,5)-decan
(21) 2,2,7,7,9,9-Hexamethyl-4-benzyl-1-oxa-3-oxo-4,8-diaza-spiro-(4,5)-decan
(22) 1',6'-Bis-[2,2,7,7,9,9-hexamethyl-1-oxa-3-oxo-4,8-diaza-spiro-(4,5)-decyl-4-]-hexan
(23) 1',10'-Bis-[2,2,7,7,9,9-hexamethyl-1-oxa-3-oxo-4,8-diaza-spiro-(4,5)-decyl-4-]-decan
(24) 2,3,7,7,9,9-Hexamethyl-2-ethyl-1-oxa-3,8-diaza-4-oxo-spiro-(4,5)-decan
(25) 2,7,7,9,9-Pentamethyl-2-ethyl-3-hexyl-1-oxa-3,8-diaza-4-oxo-spiro-(4,5)-decan
(26) 2,7,7,9,9-Pentamethyl-2-ethyl-3-octadecyl-1-oxa-3,8-diaza-4-oxo-spiro-(4,5)-decan
(27) 1',6'-Bis-[2,7,7,9,9-pentamethyl-2-ethyl-1-oxa-3,8-diaza-4-oxo-spiro-(4,5)-decyl-3-]-hexan
(28) 2,4,7,7,9,9-Hexamethyl-2-ethyl-1-oxa-3-oxo-4,8-diaza-spiro-(4,5)-decan
(29) 2,7,7,9,9-Pentamethyl-2-ethyl-4-hexyl-1-oxa-3-oxo-4,8-diaza-spiro-(4,5)-decan
(30) 2,7,7,9,9-Pentamethyl-2-ethyl-4-benzyl-1-oxa-3-oxo-4,8-diaza-spiro-(4,5)-decan
(31) 1',4'-Bis-[2,7,7,9,9-pentamethyl-2-ethyl-1-oxa-3-oxo-4,8-diaza-spiro-(4,5)-decyl-4-]-butan
(32) 2,3,7,7,9,9-Hexamethyl-2-iso-propyl-1-oxa-3,8-diaza-4-oxo-spiro-(4,5)-decan
(33) 2,7,7,9,9-Pentamethyl-2-propyl-3-ethyl-1-oxa-3,8-diaza-4-oxo-spiro-(4,5)-decan
(34) 1',4'-Bis-[2,7,7,9,9-pentamethyl-2-propyl-1-oxa-3,8-diaza-4-oxo-spiro-(4,5)-decyl-3-]-butan
(35) 1',10'-Bis-[2,7,7,9,9-pentamethyl-2-propyl-1-oxa-3,8-diaza-4-oxo-spiro-(4,5)-decyl-3-]-decan
(36) 2,7,7,9,9-Pentamethyl-2-iso-propyl-4-hexyl-1-oxa-3-oxo-4,8-diaza-spiro-(4,5)-decan
(37) 2,4,7,7,9,9-Hexamethyl-2-iso-propyl-1-oxa-3-oxo-4,8-diaza-spiro-(4,5)-decan
(38) 2,7,7,9,9-Pentamethyl-2-iso-propyl-4-ethyl-1-oxa-3-oxo-4,8-diaza-spiro-(4,5)-decan
(39) 2,3,7,7,9,9-Hexamethyl-2-iso-butyl-3,8-diaza-1-oxa-4-oxo-spiro-(4,5)-decan
(40) 2,7,7,9,9-Pentamethyl-2-iso-butyl-3-ethyl-1-oxa-3,8-diaza-4-oxo-spiro-(4,5)-decan
(41) 2,7,7,9,9-Pentamethyl-2-iso-butyl-3-butyl-1-oxa-3,8-diaza-4-oxo-spiro-(4,5)-decan
(42) 2,7,7,9,9-Pentamethyl-2-iso-butyl-3-hexyl-1-oxa-3,8-diaza-4-oxo-spiro-(4,5)-decan

(43) 2,7,7,9,9-Pentamethyl-2-iso-butyl-3-nonyl-1-oxa-3,8-diaza-4-oxo-spiro-(4,5)-decan
(44) 2,7,7,9,9-Pentamethyl-2-iso-butyl-3-octadecyl-1-oxa-3,8-diaza-4-oxo-spiro-(4,5)-decan
(45) 1',6'-Bis-[2,7,7,9,9-pentamethyl-2-iso-butyl-1-oxa-3,8-diaza-4-oxo-spiro-(4,5)-decyl-3-]-hexan
(46) 1',10'-Bis-[2,7,7,9,9-pentamethyl-2-iso-butyl-1-oxa-3,8-diaza-4-oxo-spiro-(4,5)-decyl-3-]-decan
(47) 2,7,7,9,9-Pentamethyl-2-butyl-4-ethyl-1-oxa-3-oxo-4,8-diaza-spiro-(4,5)-decan
(48) 2,7,7,8,9,9-Hexamethyl-2-iso-butyl-4-hexyl-1-oxa-3-oxo-4,8-diaza-spiro-(4,5)-decan
(49) 2,7,7,8,9,9-Hexamethyl-2-iso-butyl-4-octadecyl-1-oxa-3-oxo-4,8-diaza-spiro-(4,5)-decan
(50) 2,7,7,9,9-Pentamethyl-2-pentyl-3-benzyl-1-oxa-3,8-diaza-4-oxo-spiro-(4,5)-decan
(51) 2,4,7,7,9,9-Hexamethyl-2-pentyl-1-oxa-3-oxo-4,8-diaza-spiro-(4,5)-decan
(52) 2,7,7,9,9-Pentamethyl-2-pentyl-4-hexyl-1-oxa-3-oxo-4,8-diaza-spiro-(4,5)-decan
(53) 2,7,7,9,9-Pentamethyl-2-pentyl-4-benzyl-1-oxa-3-oxo-4,8-diaza-spiro-(4,5)-decan
(54) 2,7,7,9,9-Pentamethyl-2-pentyl-4-octadecyl-1-oxa-3-oxo-4,8-diaza-spiro-(4,5)-decan
(55) 1',4'-Bis-[2,7,7,9,9-pentamethyl-2-pentyl-1-oxa-3-oxo-4,8-diaza-spiro-(4,5)-decyl-4-]-butan
(56) 2,7,7,9,9-Pentamethyl-2-hexyl-3-nonyl-1-oxa-3,8-diaza-4-oxo-spiro-(4,5)-decan
(57) 2,4,7,7,9,9-Hexamethyl-2-hexyl-1-oxa-3-oxo-4,8-diaza-spiro-(4,5)-decan
(58) 2,7,7,9,9-Pentamethyl-2-hexyl-4-ethyl-1-oxa-3-oxo-4,8-diaza-spiro-(4,5)-decan
(59) 2,7,7,9,9-Pentamethyl-2-hexyl-4-butyl-1-oxa-3-oxo-4,8-diaza-spiro-(4,5)-decan
(60) 2,7,7,9,9-Pentamethyl-2,4-dihexyl-1-oxa-3-oxo-4,8-diaza-spiro-(4,5)-decan
(61) 1',4'-Bis-[2,7,7,9,9-pentamethyl-2-hexyl-1-oxa-3-oxo-4,8-diaza-spiro-(4,5)-decyl-4-]-butan
(62) 1',6'-Bis-[2,7,7,9,9-pentamethyl-2-hexyl-1-oxa-3-oxo-4,8-diaza-spiro-(4,5)-decyl-4-]-hexan
(63) 2,7,7,9,9-Pentamethyl-2-hexyl-4-octadecyl-1-oxa-3-oxo-4,8-diaza-spiro-(4,5)-decan
(64) 2,7,7,9,9-Pentamethyl-2-iso-hexyl-4-benzyl-1-oxa-3-oxo-4,8-diaza-spiro-(4,5)-decan
(65) 2,7,7,9,9-Pentamethyl-2-nonyl-3-ethyl-1-oxa-3,8-diaza-4-oxo-spiro-(4,5)-decan
(66) 2,7,7,9,9-Pentamethyl-2-nonyl-3-butyl-1-oxa-3,8-diaza-4-oxo-spiro-(4,5)-decan
(67) 2,7,7,9,9-Pentamethyl-2-nonyl-3-hexyl-1-oxa-3,8-diaza-4-oxo-spiro-(4,5)-decan
(68) 2,7,7,9,9-Pentamethyl-2-nonyl-3-octadecyl-1-oxa-3,8-diaza-4-oxo-spiro-(4,5)-decan
(69) 1',6'-Bis-[2,7,7,9,9-pentamethyl-2-nonyl-1-oxa-3,8-diaza-4-oxo-spiro-(4,5)-decyl-3-]-hexan
(70) 2,3,7,7,9,9-Hexamethyl-2-undecyl-1-oxa-3,8-diaza-4-oxo-spiro-(4,5)-decan
(71) 2,7,7,9,9-Pentamethyl-2-undecyl-3-ethyl-1-oxa-3,8-diaza-4-oxo-spiro-(4,5)-decan
(72) 2,7,7,9,9-Pentamethyl-2-undecyl-3-hexyl-1-oxa-3,8-diaza-4-oxo-spiro-(4,5)-decan
(73) 2,7,7,9,9-Pentamethyl-2-undecyl-3-octadecyl-1-oxa-3,8-diaza-4-oxo-spiro-(4,5)-decan
(74) 2,7,7,9,9-Pentamethyl-2-undecyl-3-benzyl-1-oxa-3,8-diaza-4-oxo-spiro-(4,5)-decan
(75) 2,7,7,9,9-Pentamethyl-2-undecyl-3-cumylyl-1-oxa-3,8-diaza-4-oxo-spiro-(4,5)-decan
(76) 1',6'-Bis-[2,7,7,9,9-pentamethyl-2-undecyl-1-oxa-3,8-diaza-4-oxo-spiro-(4,5)-decyl-3-]-hexan
(77) 1',10'-Bis-[2,7,7,9,9-pentamethyl-2-undecyl-1-oxa-3,8-diaza-4-oxo-spiro-(4,5)-decyl-3-]-decan
(78) 2,7,7,9,9-Pentamethyl-2-undecyl-1-oxa-3-oxo-4-ethyl-4,8-diaza-spiro-(4,5)-decan
(79) 2,7,7,9,9-Pentamethyl-2-undecyl-1-oxa-3-oxo-4-benzyl-4,8-diaza-spiro-(4,5)-decan
(80) 2,7,7,9,9-Pentamethyl-2-undecyl-1-oxa-3-oxo-4-octadecyl-4,8-diaza-spiro-(4,5)-decan
(81) 1',6'-Bis-[2,7,7,9,9-pentamethyl-2-undecyl-1-oxa-3-oxo-4,8-diaza-spiro-(4,5)-decyl-4-]-hexan
(82) 2,3,7,7,9,9-Hexamethyl-2-octadecyl-1-oxa-3,8-diaza-4-oxo-spiro-(4,5)-decan
(83) 2,7,7,9,9-Pentamethyl-2-octadecyl-3-ethyl-1-oxa-3,8-diaza-4-oxo-spiro-(4,5)-decan
(84) 2,7,7,9,9-Pentamethyl-2-octadecyl-3-hexyl-1-oxa-3,8-diaza-4-oxo-spiro-(4,5)-decan
(85) 2,7,7,9,9-Pentamethyl-2,3-dioctadecyl-1-oxa-3,8-diaza-4-oxo-spiro-(4,5)-decan
(86) 1',6'-Bis-[2,7,7,9,9-pentamethyl-2-octadecyl-1-oxa-3,8-diaza-4-oxo-spiro-(4,5)-decyl-3-]-hexan
(87) 1',10'-Bis-[2,7,7,9,9-pentamethyl-2-octadecyl-1-oxa-3,8-diaza-4-oxo-spiro-(4,5)-decyl-3-]-decan
(88) 3,7,7,9,9-Pentamethyl-2,2-diethyl-1-oxa-3,8-diaza-4-oxo-spiro(4,5)-decan
(89) 7,7,9,9-Tetramethyl-2,2,3-triethyl-1-oxa-3,8-diaza-4-oxo-spiro-(4,5)-decan
(90) 7,7,9,9-Tetramethyl-2,2-diethyl-3-butyl-1-oxa-3,8-diaza-4-oxo-spiro-(4,5)-decan
(91) 7,7,9,9-Tetramethyl-2,2-diethyl-3-hexyl-1-oxa-3,8-diaza-4-oxo-spiro-(4,5)-decan
(92) 7,7,9,9-Tetramethyl-2,2-diethyl-3-benzyl-1-oxa-3,8-diaza-4-oxo-spiro-(4,5)-decan
(93) 1',6'-Bis-[7,7,9,9-tetramethyl-2,2-diethyl-1-oxa-3,8-diaza-4-oxo-spiro-(4,5)-decyl-3-]-hexan
(94) 3,7,7,9,9-Pentamethyl-2-ethyl-2-iso-butyl-1-oxa-3,8-diaza-4-oxo-spiro-(4,5)-decan
(95) 7,7,9,9-Tetramethyl-2-ethyl-2-iso-butyl-3-hexyl-1-oxa-3,8-diaza-4-oxo-spiro-(4,5)-decan
(96) 7,7,9,9-Tetramethyl-2-ethyl-2-iso-butyl-3-benzyl-1-oxa-3,8-diaza-4-oxo-spiro-(4,5)-decan
(97) 7,7,9,9-Tetramethyl-2-ethyl-2-butyl-1-oxa-3-oxo-4-benzyl-4,8-diaza-spiro-(4,5)-decan
(98) 1',4'-Bis-[7,7,9,9-tetramethyl-2-ethyl-2-butyl-1-oxa-3-oxo-4,8-diaza-spiro-(4,5)-decyl-4-]-butan
(99) 7,7,9,9-Pentamethyl-2,2-dibutyl-4-benzyl-1-oxa-4,8-diaza-3-oxo-spiro-(4,5)-decan
(100) 7,7,9,9-Tetramethyl-2,2-dipentyl-3-hexyl-1-oxa-3,8-diaza-4-oxo-spiro-(4,5)-decan
(101) 7,7,9,9-Tetramethyl-2,2-dipentyl-3-octadecyl-1-oxa-3,8-diaza-4-oxo-spiro-(4,5)-decan
(102) 7,7,9,9-Tetramethyl-2,2-dipentyl-3-benzyl-1-oxa-3,8-diaza-4-oxo-spiro-(4,5)-decan

(103) 1',4'-Bis-[7,7,9,9-tetramethyl-2,2-dipentyl-1-oxa-3,8-diaza-4-oxo-spiro-(4,5)-decyl-3-]-butan
(104) 1',6'-Bis-[7,7,9,9-tetramethyl-2,2-dipentyl-1-oxa-3,8-diaza-4-oxo-spiro-(4,5)-decyl-3-]-hexan
(105) 1',10'-Bis-[7,7,9,9-tetramethyl-2,2-dipentyl-1-oxa-3,8-diaza-4-oxo-spiro-(4,5)-decyl-3-]-decan
(106) 7,7,9,9-Tetramethyl-2,2-ditetradecyl-3-hexyl-1-oxa-3,8-diaza-4-oxo-spiro-(4,5)-decan
(107) 1',6'-Bis-[7,7,9,9-tetramethyl-2,2-ditetradecyl-1-oxa-3,8-diaza-4-oxo-spiro-(4,5)-decyl-3-]-hexan
(108) 7,7,9,9-Tetramethyl-2,2-dioctadecyl-3-benzyl-1-oxa-3,8-diaza-4-oxo-spiro-(4,5)-decan
(109) 7,7,9,9-Tetramethyl-2,2-dioctadecyl-3-hexyl-1-oxa-3,8-diaza-4-oxo-spiro-(4,5)-decan
(110) 7,7,9,9-Tetramethyl-2,2,3-trioctadecyl-1-oxa-3,8-diaza-4-oxo-spiro-(4,5)-decan
(111) 1',6'-Bis-[7,7,9,9-tetramethyl-2,2-dioctadecyl-1-oxa-3,8-diaza-4-oxo-spiro-(4,5)-decyl-3-]-hexan
(112) 3,7,7,9,9-Pentamethyl-2-phenyl-1-oxa-3,8-diaza-4-oxo-spiro-(4,5)-decan
(113) 7,7,9,9-Tetramethyl-2-iso-propyl-4-ethyl-1-oxa-4,8-diaza-3-oxo-spiro-(4,5)-decan
(114) 7,7,9,9-Tetramethyl-2-iso-hexyl-3-ethyl-1-oxa-3,8-diaza-4-oxo-spiro-(4,5)-decan
(115) 3,7,7,9,9-Pentamethyl-2-nonyl-1-oxa-3,8-diaza-4-oxo-spiro-(4,5)-decan
(116) 7,7,9,9-Tetramethyl-2-iso-nonyl-3-hexyl-1-oxa-3,8-diaza-4-oxo-spiro-(4,5)-decan
(117) 7,7,9,9-Tetramethyl-2-iso-heptyl-3-octadecyl-1-oxa-3,8-diaza-4-oxo-spiro-(4,5)-decan
(118) 7,7,9,9-Tetramethyl-2-undecyl-3-benzyl-1-oxa-3,8-diaza-4-oxo-spiro-(4,5)-decan
(119) 1',4'-Bis-[7,7,9,9-tetramethyl-2-iso-nonyl-1-oxy-3,8-diaza-4-oxo-spiro-(4,5)-decyl-3-]-butan
(120) 4,7,7,9,9-Pentamethyl-2-iso-butyl-1-oxa-3-oxo-4,8-diaza-spiro-(4,5)-decan
(121) 7,7,9,9-Tetramethyl-2-iso-heptyl-1-oxa-4-ethyl-3-oxo-4,8-diaza-spiro-(4,5)-decan
(122) 1',5'-Bis-[7,7,9,9-tetramethyl-2-iso-pentyl-1-oxa-3-oxo-4,8-diaza-spiro-(4,5)-decyl-4-]-pentan
(123) 2,2,4,4-Tetramethyl-13-hexyl-7-oxa-3,13-diaza-14-oxo-dispiro-(5,1,4,2)-tetradecan
(124) 2,2,4,4-Tetramethyl-13-ethyl-7-oxa-3,13-diaza-24-oxo-dispiro-(5,1,4,2)-tetradecan
(125) 2,2,4,4-Tetramethyl-13-butyl-7-oxa-3,13-diaza-14-oxo-dispiro-(5,1,4,2)-tetradecan
(126) 2,2,4,4-Tetramethyl-14-ethyl-7-oxa-3,14-diaza-15-oxo-dispiro-(5,1,5,2)-pentadecan
(127) 2,2,4,4-Tetramethyl-14-butyl-7-oxa-3,14-diaza-15-oxo-dispiro-(5,1,5,2)-pentadecan
(128) 2,2,4,4-Tetramethyl-14-hexyl-7-oxa-3,14-diaza-15-oxo-dispiro-(5,1,5,2)-pentadecan
(129) 2,2,4,4-Tetramethyl-14-benzyl-7-oxa-3,14-diaza-15-oxo-dispiro-(5,1,5,2)-pentadecan
(130) 1',4'-Bis-[2,2,4,4-tetramethyl-7-oxa-3,14-diaza-15-oxo-dispiro-(5,1,5,2)-pentadecyl-14]-butan
(131) 1',4'-Bis-[2,2,4,4-tetramethyl-1-oxa-3,14-diaza-15-oxo-dispiro-(5,1,5,2)-pentadecyl-14]-butan
(132) 1',6'-Bis-[2,2,4,4-tetramethyl-7-oxa-3,14-diaza-15-oxo-dispiro-(5,1,5,2)-pentadecyl-14]-hexan
(133) 2,2,4,4,10,12-Hexamethyl-14-ethyl-7-oxa-3,14-diaza-15-oxo-dispiro-(5,1,5,2)-pentadecan
(134) 2,2,4,4,10,10,12,12-Octamethyl-14-ethyl-7-oxa-3,14-diaza-15-oxo-dispiro-(5,1,5,2)-pentadecan
(135) 2,2,4,4,15-Pentamethyl-7-oxa-3,15-diaza-14-oxo-dispiro-(5,1,5,2)-pentadecan
(136) 2,2,4,4-Tetramethyl-15-hexyl-7-oxa-3,15-diaza-14-oxo-dispiro-(5,1,5,2)-pentadecan
(137) 2,2,4,4,20-Pentamethyl-7-oxa-3,20-diaza-21-oxo-dispiro-(5,1,11,2)-heneicosan
(138) 2,2,4,4-Tetramethyl-20-ethyl-7-oxa-3,20-diaza-21-oxo-dispiro-(5,1,11,2)-heneicosan
(139) 2,2,4,4-Tetramethyl-20-iso-propyl-7-oxa-3,20-diaza-21-oxo-dispiro-(5,1,11,2)-heneicosan
(140) 2,2,4,4-Tetramethyl-20-butyl-7-oxa-3,20-diaza-21-oxo-dispiro-(5,1,11,2)-heneicosan
(141) 2,2,4,4-Tetramethyl-20-iso-butyl-7-oxa-3,20-diaza-21-oxo-dispiro-(5,1,11,2)-heneicosan
(142) 2,2,4,4-Tetramethyl-20-pentyl-7-oxa-3,20-diaza-21-oxo-dispiro-(5,1,11,2)-heneicosan
(143) 2,2,4,4-Tetramethyl-20-hexyl-7-oxa-3,20-diaza-21-oxo-dispiro-(5,1,11,2)-heneicosan
(144) 2,2,4,4-Tetramethyl-20-octyl-7-oxa-3,20-diaza-21-oxo-dispiro-(5,1,11,2)-heneicosan
(145) 2,2,4,4-Tetramethyl-20-heptyl-7-oxa-3,20-diaza-21-oxo-dispiro-(5,1,11,2)-heneicosan
(146) 2,2,4,4-Tetramethyl-20-octadecyl-7-oxa-3,20-diaza-21-oxo-dispiro-(5,1,11,2)-heneicosan
(147) 2,2,4,4-Tetramethyl-20-benzyl-7-oxa-3,20-diaza-21-oxo-dispiro-(5,1,11,2)-heneicosan
(148) 1',4'-Bis-[2,2,4,4-tetramethyl-7-oxa-3,20-diaza-21-oxo-dispiro-(5,1,11,2)-heneicosyl-20-]-butan
(149) 1',5'-Bis-[2,2,4,4-tetramethyl-7-oxa-3,20-diaza-21-oxo-dispiro-(5,1,11,2)-heneicosyl-20-]-pentan
(150) 1',6'-Bis-[2,2,4,4-tetramethyl-7-oxa-3,20-diaza-21-oxo-dispiro-(5,1,11,2)-heneicosyl-20-]-hexan
(151) 1',10'-Bis-[2,2,4,4-tetramethyl-7-oxa-3,20-diaza-21-oxo-dispiro-(5,1,11,2)-heneicosyl-20-]-decan
(152) 2,2,4,4,21-Pentamethyl-7-oxa-3,21-diaza-20-oxo-dispiro-(5,1,11,2)-heneicosan
(153) 7,7,9,9-Tetramethyl-2,2-dibenzyl-3-hexyl-1-oxa-3,8-diaza-4-oxo-spiro-(4,5)-decan
(154) 7,7,9,9-Tetramethyl-2,2-dibenzyl-3-octadecyl-1-oxa-3,8-diaza-4-oxo-spiro-(4,5)-decan
(155) 7,7,9,9-Tetramethyl-2,2,3-tribenzyl-1-oxa-3,8-diaza-4-oxo-spiro-(4,5)-decan

(156) 1,2-Bis-[2,2,7,7,9,9-hexamethyl-1-oxa-3,8-diaza-4-oxo-dispiro-(4,5)-decen-3-methyl-]-benzol

Die neuen Verbindungen werden aus Polyalkyl-1-oxa-diaza-spiro-decanen der Formel (IV), welche ihrerseits nach den Vorschriften der DE-PS 2 606 026, 2 634 957 und 2 834 962 zugänglich sind, und Halogenverbindungen der Formel (V) unter Halogenwasserstoffabspaltung gemäß der folgenden Reaktionsgleichung erhalten.

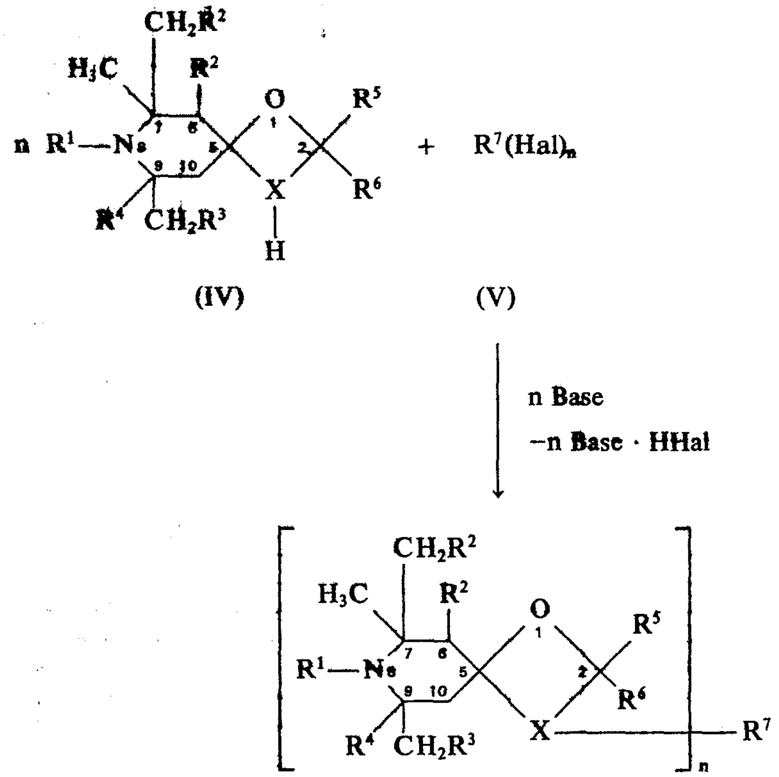


(IV) (V)

(I)

Die Reste $R^1$, $R^2$, $R^3$, $R^4$, $R^5$, $R^6$, $R^7$ und X haben die oben angegebene Bedeutung. Hal bedeutet Chlor, Brom oder Jod, n ist 1 oder 2.

Im einzelnen kommen beispielsweise folgende Reste in Frage:

$R^1$ = Wasserstoff, Sauerstoff, Methyl, Butyl, Hexyl, Octyl, Dodecyl
$R^2$ = Wasserstoff, Methyl, Ethyl, Butyl
$R^3$ = Wasserstoff, Methyl, Ethyl, Butyl
$R^4$ = Methyl
$R^3$ und $R^4$ zusammen mit dem C-Atom 9 = Cyclohexyl, 2,2,6,6-Tetramethylpiperidyl
$R^5$ und $R^6$ = Wasserstoff, Ethyl, Propyl, iso-Propyl, Butyl, iso-Butyl, Pentyl, iso-Pentyl, Hexyl, iso-Hexyl, Heptyl, iso-Heptyl, Octyl, Nonyl, iso-Nonyl, Decyl, Undecyl, Tridecyl, Octadecyl, Phenyl, 2,4-Dichlorphenyl
$R^5$ und $R^6$ zusammen mit dem C-Atom 2 = Cyclohexyliden, 3,5-Dimethylcyclohexyliden, 3,3,5,5-Tetramethylcyclohexyliden, Cycloheptyliden, Cyclododecyliden, 2,2,6,6-Tetramethylpiperidyliden
$R^7$ = Methyl, Ethyl, Propyl, Butyl, Pentyl, Hexyl, Heptyl, Octyl, Nonyl, Dodecyl, Tetradecyl, Octadecyl, Benzyl, Ethylen, Propylen, Butylen, Pentylen, Hexylen, Decylen, Butenylen, Xylylen

Bei der Synthese geht man so vor, daß man im Falle von n = 1 die Verbindungen (IV) und (V) im Molverhältnis 1 : 1 bis 1 : 10, vorzugsweise 1 : 1,2 bis 1 : 5 und insbesondere 1 : 1,5 bis 1 : 3 und im Falle von n = 2 im Molverhältnis 2,5 : 1 bis 2 : 1, vorzugsweise 2,1 : 1 bis 2 : 1 und insbesondere 2 : 1 miteinander umsetzt. Man arbeitet in einem organischen Lösungsmittel in Gegenwart von äquimolaren bis 20fach molaren Mengen festen Alkalimetallhydroxids oder auch einer 20- bis bevorzugt 50%igen wäßrigen Alkalimetallhydroxidlösung bei Anwesenheit eines Phasentransferkatalysators. Die Reaktionstemperatur kann im Bereich von 20 bis 120, vorzugsweise 20 bis 70 und insbesondere 40 bis 60°C gewählt werden. Als organisches Löungsmittel verwendet man aliphatische oder aromatische Kohlenwasserstoffe, wie z. B. Petrolether, Hexan, Heptan, Benzinschnitte, Toluol, Cyclohexan etc. oder einen Überschuß an Alkylhalogenid. Unter Phasentransferkatalysatoren werden Verbindungen aus der Gruppe der quartären Ammoniumhalogenide verstanden, die man in Mengen von 0,1 bis 5

Gew.-%, bezogen auf Verbindung (IV), einsetzt. Besonders gut geeignet ist Tricaprylmethylammoniumchlorid. Die Reaktion ist im allgemeinen nach 1 bis 20 Stunden, je nach Reaktionsfähigkeit der Halogenkomponente, beendet.

Zur Aufarbeitung werden die Phasen, gegebenenfalls nach Zugabe von wenig Wasser, getrennt. Die organische Phase wird mehrfach mit Wasser gewaschen, über $Na_2SO_4$ oder $MgSO_4$ getrocknet, eingeengt und der Rückstand umkristallisiert. In einigen Fällen kristallisieren die Endprodukte bereits aus den Reaktionslösungen aus und werden dann durch Filtration gewonnen.

Es war überraschend und nicht vorherzusehen, daß es möglich sein würde, insbesondere die Verbindungen, in denen $R^1 = H$ ist, nach der vorstehend geschilderten Methode herzustellen. Im Gegensatz zum tatsächlich beobachteten und durch $^{13}C$-NMR-Untersuchungen bewiesenen Reaktionsverlauf hätte nämlich eher mit einer Substitution am Piperidinstickstoff gerechnet werden müssen, denn dieser besitzt aufgrund seiner höheren Basizität eine viel größere nucleophile Kraft als der Amidstickstoff in der Atomgruppe X.

Was die Eigenschaften und Wirksamkeit der neuen Verbindungen betrifft, so wäre eigentlich zu erwarten gewesen, daß im Vergleich zu ähnlich strukturierten 1-Oxa-diaza-spirodecanen, die in der Patentliteratur bereits beschrieben und als Kunststoffstabilisatoren empfohlen worden sind (DE-AA 1 770 689 und 2 227 689), keine oder nur geringfügige Unterschiede bestehen. Überraschenderweise sind die neuen Verbindungen jedoch auch der besten der in den obengenannten DE-AA aufgeführten Verbindungen, das ist die des Beispiels 58 der DE-A-2 227 689, erheblich überlegen. Diese Verbindung — es ist das 7,7,8,9,9-Pentamethyl-3-epoxypropyl-1,3,8-triaza-spiro-(4,5)-decan-2,4-dion — zeichnet sich zwar durch die höchste Stabilisatorwirksamkeit aller dort beschriebenen Verbindungen aus, nachteilig ist jedoch ihre zu hohe Flüchtigkeit bei der Verarbeitung, was auch für die Verbindungen der DE-AA 2 606 026 und 2 634 957 zutrifft, und ihre zu gute Auswaschbarkeit aus den sie enthaltenden Thermoplasten. Demgegenüber sind die ebenfalls in der DE-A-2 227 689 beschriebenen Verbindungen mit höheren Molgewichten (z. B. die Verbindung Nr. 104), die den erfindungsgemäßen Stabilisatoren strukturell verwandt sind, von ihrer Wirkung her gesehen schlechter als Verbindung 58 (vgl. die Beispiele 17 und 18) und damit als Stabilisatoren für Kunststoffe völlig unzureichend. Von den Ausgangs-Spirodecanen zur Herstellung der erfindungsgemäßen Verbindungen (DE-AA 2 606 026 und 2 634 957) sind schließlich aus der EP-A-0 001 207 auch Harnstoffderivate bekannt, deren Thermostabilität den gestiegenen Anforderungen jedoch nicht mehr genügt.

An hochwertige Stabilisatoren werden nun aber zusätzlich zur stabilisierenden Wirkung gerade bezüglich der genannten physikalischen Eigenschaften hohe Anforderungen gestellt, denn eine zu hohe Flüchtigkeit führt zu beträchtlichen Stabilisatorverlusten bei der Verarbeitung der Polymeren und eine zu gute Auswaschbarkeit, z. B. bei der Beregnung mit Wasser, vermindert die Wirksamkeit des Stabilisators.

Daß die erfindungsgemäßen Verbindungen trotz ihrer Verwandtschaft mit den Verbindungen der zitierten Offenlegungsschriften nicht nur als Lichtschutzmittel besser wirksam sind, sondern sich darüber hinaus durch besonders niedrige Flüchtigkeit und geringe Auswaschbarkeit auszeichnen — wobei diese vorteilhaften Eigenschaften in besonderem Maße die Verbindungen mit $n = 2$ besitzen —, war nicht vorhersehbar und muß als erheblicher technischer Fortschritt angesehen werden.

Wie bereits erwähnt, werden die neuen Verbindungen als Stabilisatoren für Kunststoffe gegen deren Schädigung durch Einwirkung von Sauerstoff, Wärme und Licht verwendet. Beispiele für solche Kunststoffe sind:

Polymere, die sich von einfach oder doppelt ungesättigten Kohlenwasserstoffen ableiten, z. B. Polyolefine wie Polyethylen, das gegebenenfalls vernetzt sein kann, Polypropylen, Polybuten-1, Polyisobuten, Polymethylbuten-1, Polymethylpenten-1, Polyisopren, Polybutadien, Polystyrol, Copolymere der den genannten Homopolymeren zugrunde liegenden Monomeren, wie Ethylen-Propylen-Copolymere, Propylen-Buten-1-Copolymere, Propylen-Isobuten-Copolymere, Styrol-Butadien-Copolymere sowie Terpolymere und Ethylen und Propylen mit einem Dien, wie z. B. Hexadien, Dicyclopentadien oder Ethylidennorbornen; Mischungen der obengenannten Homopolymeren, wie beispielsweise Gemische von Polypropylen und Polyethylen, Polypropylen und Polybuten-1, Polypropylen und Polyisobutylen oder von Butadien-Acrylnitril-Copolymerisat mit einem Styrol-Butadien-Copolymerisat.

Halogenhaltige Vinylpolymere wie Polyvinylchlorid, Polyvinylidenchlorid, Polyvinylfluorid, Polychloropren und Chlorkautschuke, sowie Copolymere von Vinylchlorid und Vinylidenchlorid untereinander und mit anderen olefinisch ungesättigten Monomeren

Polymere, die sich von $\alpha,\beta$-ungesättigten Säuren und deren Derivaten ableiten, wie Polyacrylate und Polymethacrylate, Polyacrylamide und Polyacrylnitril sowie deren Copolymere untereinander und mit anderen Vinylverbindungen, wie Acrylnitril-Butadien-Styrol-, Acrylnitril-Styrol- und Acrylnitril-Styrol-Acrylester-Copolymerisate

Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin und deren Copolymere mit anderen

Vinylverbindungen, wie Ethylen-Vinylacetat-Copolymere
Mono- und Copolymere, die sich von Epoxiden ableiten, wie Polyethylenoxid oder die Polymerisate, die sich von Bisglycidylethern ableiten
Polyacetale, wie Polyoximethylen und Polyoxiethylen, sowie solche Polyoximethylene, die als Comonomeres Ethylenoxid enthalten
Polyurethane und Polyharnstoffe
Polycarbonate
Polyamide und Copolyamide, die sich von Diaminen und Dicarbonsäuren und/oder von Aminocarbonsäuren oder den entsprechenden Lactamen ableiten, wie Polyamid 6, Polyamid 6/6, Polyamid 6/10, Polyamid 11, Polyamid 12
Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylol-cyclohexanterephthalat
Vernetzte Polymerisate, die sich von Aldehyden einerseits und Phenolen, Harnstoffen und Melaminen andererseits ableiten, wie Phenol-Formaldehyd-, Harnstoff-Formaldehyd- und Melamin-Formaldehydharze

Von besonderer Bedeutung ist die Stabilisierung von Polyolefinen, Styrolpolymerisaten, Polyamiden, Poly(meth-)acrylaten und von Polyurethanen, für die sich die neuen Verbindungen hervorragend eignen. Beispiele hierfür sind Polyethylen hoher und niedriger Dichte, Polypropylen, Ethylen-Propylen-Copolymerisate, Polystyrol, Styrol-Butadien-Acrylnitril-Tercopolymerisate, Mischungen von Polyolefinen oder von Styrolpolymerisaten, Polyurethane auf Polyether- oder Polyesterbasis in Form von Lacken, Fäden, Folien, Platten, Filmen, Elastomeren oder Schaumstoffen.

Die neuen Stabilisatoren werden nach allgemein üblichen Methoden in die Polymermassen in Mengen von 0,01 bis 5, vorzugsweise 0,05 bis 2,5 und insbesondere 0,1 bis 1,0 Gew.-%, bezogen auf das zu stabilisierende Material, eingearbeitet. Die Einarbeitung kann beispielsweise durch Einmischen der Verbindungen und gegebenenfalls weiterer Additive in die Schmelze, vor oder während der Formgebung, oder auch durch Aufbringen der gelösten oder dispergierten Verbindungen auf das Polymere direkt oder Einmischen in eine Lösung, Suspension oder Emulsion desselben, gegebenenfalls unter nachträglichem Verdunsten des Lösungsmittels erfolgen. Die neuen Verbindungen können auch in Form eines Masterbatches, der diese Verbindungen beispielsweise in einer Konzentration von 2,5 bis 50, vorzugsweise 5,0 bis 20 Gew.-% enthält, den zu stabilisierenden Kunststoffen zugesetzt werden.

Die durch den Zusatz der erfindungsgemäßen Substanzen der Formel (I) stabilisierten Kunststoffe können gegebenenfalls noch andere bekannte und übliche Zusätze, wie beispielsweise Anitoxidantien auf Phenol- und Sulfidbasis, UV-Absorber und Lichtschutzmittel, Phosphitstabilisatoren, Metallverbindungen, Epoxistabilisatoren und mehrwertige Alkohole enthalten.

Beispiele für Antioxidantien sind solche vom Typ der sterisch gehinderten Phenole wie 4,4'-Butyliden-bis(2,6-di-tert.-butylphenol), 4,4'-Thio-bis-(2-tert.-butyl-5-methylphenol), phenolische Triazinverbindungen, Thiodipropionsäureester von Fettalkoholen, Dioctadecylsulfid und -disulfid.

Zu den UV-Absorbern und Lichtschutzmitteln gehören z. B. 2-(2'-Hydroxyphenyl)-benztriazole wie 2-(2'-Hydroxy-5'-methyl-phenyl)-benztriazol, 2-Hydroxybenzophenone wie 2-Hydroxy-4-octoxy-benzophenol, Stabilisatoren aus der Gruppe der Salizylate wie Octylphenylsalizylat, Nickelchelate, Oxalsäurediamide und sterisch gehinderte Piperidinverbindungen.

Als Phosphite sind Trisnonylphenylphosphit, Trislaurylphosphit oder auch Ester des Pentaerythritphosphits zu nennen.

Unter als Stabilisatoren bekannten Metallverbindungen werden in diesem Zusammenhang verstanden: Calcium-, Barium-, Strontium-, Zink-, Cadmium-, Magnesium-, Aluminium- und Bleiseifen aliphatischer Carbonsäuren oder Oxycarbonsäuren mit etwa 12 bis 32 C-Atomen, Salze der genannten Metalle mit aromatischen Carbonsäuren wie Benzoate oder Salizylate sowie (Alkyl-)Phenolate dieser Metalle, ferner Organozinnverbindungen, wie z. B. Dialkylzinnthioglykolate und Carboxylate.

Bekannte Epoxistabilisatoren sind z. B. epoxidierte höhere Fettsäuren wie epoxidiertes Sojabohnenöl, Tallöl, Leinöl oder epoxidiertes Butyloleat sowie Epoxide langkettiger Olefine.

Mehrwertige Alkohole können beispielsweise Pentaerythrit, Trimethylolpropan, Sorbit oder Mannit sein, d. h. bevorzugt Alkohole mit 5 oder 6 C-Atomen und 2 bis 6 OH-Gruppen.

Eine wirksame Stabilisatorkombination für Poly-$\alpha$-Olefine, wie z. B. Hoch-, Mittel- und Niederdruckpolymerisate von $C_2$- bis $C_4$-$\alpha$-Olefinen, insbesondere Polyethylen und Polypropylen oder von Copolymerisaten derartiger $\alpha$-Olefine besteht, bezogen auf 100 Gewichtsteile Polymer, beispielsweise aus 0,01 bis 5 Gewichtsteilen einer der erfindungsgemäß zu verwendenden Verbindungen, 0,05 bis 5 Gewichtsteilen eines phenolischen Stabilisators, gegebenenfalls 0,01 bis 5 Gewichtsteilen eines schwefelhaltigen Costabilisators sowie gegebenenfalls 0,01 bis 3 Gewichtsteilen einer basischen oder neutralen Metallseife, wie z. B. Calciumstearat oder Zinkstearat sowie gegebenenfalls 0,1 bis 5 Gewichtsteilen eines Phosphits und gegebenenfalls 0,01 bis 5 Gewichtsteilen eines bekannten UV-Stabilisators aus der Gruppe der Alkoxyhydroxybenzophenone, 4-Hydroxyphenylbenzotriazole, Benzylidenmalonsäuremononitrilester oder der sog. Quencher, wie z. B. Nickelchelate.

Die erfindungsgemäß stabilisierten Kunststoffe können in verschiedenster Form angewendet werden, z. B. als Folien, Fasern, Bändchen, Profile oder als Bindemittel für Lacke, Klebemittel oder Kitte.

Die folgenden Beispiele dienen der Erläuterung des Erfindungsgegenstandes:

## Beispiel 1

2,2,4,4,7,7,9,9-Heptamethyl-1-oxa-4,8-diaza-3-oxo-spiro-(4,5)-decan (Verbindung Nr. 14)

6,8 g 2,2,7,7,9,9-Hexamethyl-1-oxa-4,8-diaza-3-oxo-spiro-(4,5)-decan und 20 g Methyljodid wurden in 50 ml Toluol vorgelegt. Nach dem Zufügen von drei Tropfen des Phasentransferkatalysators Tricaprylmethylammoniumchlorid wurde das Reaktionsgemisch mit 20 ml 50%iger Natronlauge versetzt und 5 Stunden bei 45° C gerührt. Im Verlauf der Reaktion wird der Ansatz zunächst gelartig und dann klar. Man trennt sodann die Phasen voneinander und schüttelt die organische Phase zweimal mit je 30 ml Wasser aus, filtriert und engt ein. Der Rückstand wird aus Petrolether umkristallisiert. 5,4 g weiße Kristalle/Fp. 128 bis 130° C.

Die gleichen Ergebnisse wurden beim Arbeiten in Hexan, Heptan und Cyclohexen als Lösungsmittel erhalten.

## Beispiele 2 bis 71

Es wurde analog Beispiel 1 unter Verwendung anderer Edukte gearbeitet.

In der nachstehenden Tabelle sind die Versuchsbedingungen sowie Kennzahlen der Verfahrensprodukte zusammengestellt. Spalte 2 (»Verbindung Nr.«) verweist auf die Aufstellung in der Beschreibung Seiten 3 bis 6.

| Bsp. Nr. | Verb. Nr. | Ausgangsmaterialien<br>Polyalkyl-1-oxa-diaza-spiro-decan; Menge (g) | Halogenverbindung Menge (g) | Base<br>(ml) | Reaktionszeit<br>(Stunden) | Temperatur<br>(°C) | umkrist. aus | Fp.<br>(°C) |
|---|---|---|---|---|---|---|---|---|
| 2 | 9 | 2,2,7,7,9,9-Hexamethyl-1-oxa-3,8-diaza-4-oxo-spiro-(4,5)-decan (20) | 1-Bromoctadecan (28) | 50%ige NaOH (7) | 13 | 80 | Petrolether | 117 |
| 3 | 10 | dto. (20) | Benzylchlorid (11) | dto. (7) | 16 | 80 | Heptan | 119 |
| 4 | 13 | dto. (9) | 1,10-Dibromdecan (5,7) | dto. (8) | 16 | 50 | Ether | 136–142 |
| 5 | 17 | dto. (9) | 1,6-Dibromhexan (4,6) | dto. (8) | 16 | 50 | Petrolether | 136–142 |
| 6 | 4 | dto. (9) | 1-Brombutan (20) | dto. (20) | 5 | 55 | dto. | 50–53 |
| 7 | 6 | dto. (12) | 2-Bromhexan (30) | dto. (20) | 6 | 50 | dto. | 30–33 |
| 8 | 22 | 2,2,7,7,9,9-Hexamethyl-1-oxa-3-oxo-4,8-diaza-spiro-(4,5)-decan (8,5) | 1,6-Dibromhexan (4) | KOH (2) | 24 | 55 | Hexan | 145 |
| 9 | 16 | dto. (7) | Jodethan (20) | 50%ige NaOH (20) | 6 | 55 | Heptan | 82–84 |
| 10 | 21 | dto. (6) | Benzylchlorid (30) | dto. (20) | 8 | 55 | dto. | 133–136 |
| 11 | 20 | dto. (6) | 1-Bromoctadecan (18) | dto. (20) | 16 | 55 | Petrolether | 50–52 |
| 12 | 18 | dto. (8,5) | 1-Bromhexan (30) | dto. (20) | 7 | 50 | dto. | 57–58 |
| 13 | 29 | 2,7,7,9,9-Pentamethyl-2-ethyl-1-oxa-3-oxo-4,8-diaza-spiro-(4,5)-decan (7,8) | dto. (4) | dto. (5) | 6 | 80 | Heptan | 71 |
| 14 | 30 | dto. (4,8) | Benzylchlorid (10) | dto. (5) | 6 | 60 | dto. | 108 |

Fortsetzung

| Bsp. Nr. | Verb. Nr. | Ausgangsmaterialien: Polyalkyl-1-oxa-diaza-spiro-decan; Menge (g) | Halogenverbindung Menge (g) | Base (ml) | Reaktionszeit (Stunden) | Temperatur (°C) | umkrist. aus | Fp. (°C) |
|---|---|---|---|---|---|---|---|---|
| 15 | 15 | 2,2,7,7,8,9,9-Heptamethyl-1-oxa-4,8-diaza-spiro-(4,5)-decan (7) | 1-Brombutan (25) | 50%ige NaOH (25) | 6 | 55 | Heptan | 58 |
| 16[1]) | 33 | 2,7,7,9,9-Pentamethyl-2-propyl-1-oxa-3,8-diaza-4-oxo-spiro-(4,5)-decan (3,4) | Jodethan (20) | dto. (5) | 5 | 60 | dto. | 101 |
| 17 | 34 | dto. (6,6) | 1,4-Dibrombutan (2,7) | dto. (5) | 20 | 60 | dto. | 142 |
| 18 | 36 | 2,7,7,9,9-Pentamethyl-2-iso-propyl-1-oxa-3-oxo-4,8-diaza-spiro-(4,5)-decan (8) | 1-Bromhexan (20) | dto. (20) | 6 | 60 | Hexan | 90 |
| 19 | 38 | dto. (8) | Jodethan (20) | dto. (5) | 6 | 50 | dto. | 105–107 |
| 20[2]) | 47 | 2,7,7,9,9-Pentamethyl-2-butyl-1-oxa-3-oxo-4,8-diaza-spiro-(4,5)-decan (7) | dto. | dto. (20) | 6 | 55 | Heptan | 57 |
| 21[2]) | 50 | 2,7,7,9,9-Pentamethyl-2-pentyl-1-oxa-3,8-diaza-4-oxo-spiro-(4,5)-decan (3,6) | Benzylchlorid (4) | dto. (10) | 6 | 70 | dto. | 81 |
| 22 | 53 | 2,7,7,9,9-Pentamethyl-2-pentyl-1-oxa-3-oxo-4,8-diaza-spiro-(4,5)-decan (4) | dto. (2) | dto. (5) | 6 | 65 | dto. | 56 |
| 23 | 64 | 2,7,7,9,9-Pentamethyl-2-iso-hexyl-1-oxa-3-oxo-4,8-diaza-spiro-(4,5)-decan (5,8) | dto. (10) | dto. (7) | 5 | 60 | Petrolether | 30 |

Fortsetzung

| Bsp. Nr. | Verb. Nr. | Ausgangsmaterialien<br>Polyalkyl-1-oxa-diaza-spiro-decan; Menge (g) | Halogenverbindung Menge (g) | Base (ml) | Reaktionszeit (Stunden) | Temperatur (°C) | umkrist. aus | Fp. (°C) |
|---|---|---|---|---|---|---|---|---|
| 24 | 63 | 2,7,7,9,9-Pentamethyl-2-hexyl-1-oxa-3-oxo-4,8-diaza-spiro-(4,5)-decan (6,2) | 1-Bromoctadecan (10) | 50%ige NaOH (7) | 24 | 55 | – | Wachs |
| 25 | 62 | dto. (10) | 1,6-Dibromhexan (4) | dto. (5) | 24 | 50 | Hexan | 120 |
| 26 | 61 | dto. (10) | 1,4-Dibrombutan (3,5) | dto. (8) | 24 | 50 | Heptan | 128–132 |
| 27 | 60 | dto. (8) | 1-Bromhexan (20) | dto. (20) | 8 | 60 | – | Wachs |
| 28 | 58 | dto. (8) | Bromethan (20) | dto. (20) | 6 | 40 | – | dto. |
| 29 | 59 | 2,7,7,9,9-Pentamethyl-2-hexyl-1-oxa-3-oxo-4,8-diaza-spiro-(4,5)-decan (8) | 1-Brombutan (20) | dto. (20) | 8 | 50 | – | dto. |
| 30 | 65 | 2,7,7,9,9-Pentamethyl-2-nonyl-1-oxa-3,8-diaza-4-oxo-spiro-(4,5)-decan (8) | Bromethan (20) | dto. (20) | 8 | 45 | Petrolether | 39–44 |
| 31 | 72 | 2,7,7,9,9-Pentamethyl-2-undecyl-1-oxa-3,8-diaza-4-oxo-spiro-(4,5)-decan (5) | 1-Bromhexan (20) | dto. (5) | 10 | 55 | – | Öl |
| 32[2]) | 76 | dto. (7,6) | 1,6-Dibromhexan (2,5) | dto. (10) | 8 | 60 | Petrolether | 25 |
| 33 | 79 | 2,7,7,9,9-Pentamethyl-2-undecyl-1-oxa-3-oxo-4,8-diaza-spiro-(4,5)-decan (4) | Benzylchlorid (4) | dto. (5) | 6 | 75 | dto. | 36 |

Fortsetzung

| Bsp. Nr. | Verb. Nr. | Ausgangsmaterialien Polyalkyl-1-oxa-diaza-spiro-decan; Menge (g) | Halogenverbindung Menge (g) | Base (ml) | Reaktionszeit (Stunden) | Temperatur (°C) | umkrist. aus | Fp. (°C) |
|---|---|---|---|---|---|---|---|---|
| 34[1]) | 82 | 2,7,7,9,9-Pentamethyl-2-octadecyl-1-oxa-3,8-diaza-4-oxo-spiro-(4,5)-decan (6) | Jodmethan (20) | 50%ige NaOH (8) | 6 | 40 | – | 48–50 |
| 35 | 89 | 7,7,9,9-Tetramethyl-2,2-diethyl-1-oxa-3,8-diaza-4-oxo-spiro-(4,5)-decan (6) | Bromethan (20) | dto. (20) | 8 | 50 | Petrolether | 102–105 |
| 36 | 90 | dto. (6) | 1-Brombutan (20) | dto. (20) | 8 | 50 | dto. | 85–90 |
| 37 | 91 | dto. (7) | 1-Bromhexan (20) | dto. (20) | 8 | 55 | dto. | 70–71 |
| 38 | 92 | dto. (6) | Benzylchlorid (10) | dto. (20) | 8 | 55 | dto. | 111–115 |
| 39 | 93 | dto. (6,7) | 1,6-Dibromhexan (3,3) | dto. (20) | 20 | 50 | dto. | 126 |
| 40[2]) | 97 | 7,7,9,9-Tetramethyl-2-ethyl-2-butyl-1-oxa-3-oxo-4,8-diaza-spiro-(4,5)-decan (5,0) | Benzylchlorid (5) | dto. (5) | 8 | 55 | Heptan | 75–77 |
| 41 | 99 | 7,7,9,9-Tetramethyl-2,2-dibutyl-1-oxa-3-oxo-4,8-diaza-spiro-(4,5)-decan (2) | dto. (2) | dto. (3) | 8 | 55 | dto. | 49 |
| 42 | 105 | 7,7,9,9-Tetramethyl-2,2-dipentyl-1-oxa-3,8-diaza-4-oxo-spiro-(4,5)-decan (17,6) | 1,10-Dibrompentan (7,5) | KOH (3) | 24 | 50 | Petrolether | 40–45 |
| 43 | 103 | dto. (17,6) | 1,4-Dibrombutan (5,4) | dto. (3) | 24 | 50 | dto. | 89–96 |
| 44 | 102 | dto. (5,5) | Benzylchlorid (5) | 50%ige NaOH (10) | 10 | 60 | dto. | 58 |

Fortsetzung

| Bsp. Nr. | Verb. Nr. | Ausgangsmaterialien Polyalkyl-1-oxa-diaza-spiro-decan; Menge (g) | Halogenverbindung Menge (g) | Base (ml) | Reaktionszeit (Stunden) | Temperatur (°C) | umkrist. aus | Fp. (°C) |
|---|---|---|---|---|---|---|---|---|
| 45 | 101 | 7,7,9,9-Tetramethyl-2,2-dipentyl-1-oxa-3,8-diaza-4-oxo-spiro-(4,5)-decan (5,5) | 1-Bromoctadecan (10) | 50%ige NaOH (10) | 20 | 70 | – | Wachs |
| 46 | 153 | 7,7,9,9-Tetramethyl-2,2-dibenzyl-1-oxa-3,8-diaza-4-oxo-spiro-(4,5)-decan (7) | 1-Bromhexan (15) | dto. (7) | 10 | 65 | Heptan | 122 |
| 47 | 154 | dto. (7) | 1-Bromoctadecan (15) | dto. (7) | 7 | 65 | – | Wachs |
| 48 | 155 | dto. (7) | Benzylchlorid (10) | dto. (7) | 7 | 65 | Heptan | 192 |
| 49 | 113 | 7,7,9,9-Tetramethyl-2-iso-propyl-1-oxa-3-oxo-4,8-diaza-spiro-(4,5)-decan (7,3) | Bromethan (20) | dto. (20) | 8 | 50 | Petrolether | 68–72 |
| 50[1]) | 114 | 7,7,9,9-Tetramethyl-2-iso-hexyl-1-oxa-3-oxo-4,6-diaza-spiro-(4,5)-decan (1) | Bromethan (20) | dto. (2) | 8 | 40 | dto. | 35–40 |
| 51[1]) | 123 | 2,2,4,4-Tetramethyl-7-oxa-3,13-diaza-14-oxo-dispiro-(5,1,4,2)-tetradecan (7) | 1-Bromhexan (30) | dto. (20) | 6 | 50 | dto. | 74–76 |
| 52 | 124 | dto. (8,5) | Bromethan (20) | dto. (20) | 5 | 50 | dto. | 79–81 |
| 53[3]) | 125 | dto. (8) | 1-Brombutan (20) | dto. (20) | 5 | 50 | dto. | 65–70 |

Fortsetzung

| Bsp. Nr. | Verb. Nr. | Ausgangsmaterialien Polyalkyl-1-oxa-diaza-spiro-decan; Menge (g) | Halogenverbindung Menge (g) | Base (ml) | Reaktionszeit (Stunden) | Temperatur (°C) | umkrist. aus | Fp. (°C) |
|---|---|---|---|---|---|---|---|---|
| 54[3]) | 131 | 2,2,4,4-Tetramethyl-7-oxa-3,14-diaza-15-oxo-dispiro-(5,1,5,2)-pentadecan (28) | 1,4-Dichlorbutan (6) | 50%ige NaOH (50) | 24 | 70 | Petrolether | Wachs |
| 55 | 132 | dto. (28) | 1,6-Dibromhexan (10) | dto. (50) | 24 | 70 | — | dto. |
| 56[1]) | 136 | 2,2,4,4-Tetramethyl-7-oxa-3,15-diaza-14-oxo-dispiro-(5,1,5,2)-pentadecan (7) | 1-Bromhexan (20) | dto. (20) | 5 | 50 | Petrolether | 63—65 |
| 57[1]) | 134 | 2,2,4,4,10,10,12,12-Octamethyl-7-oxa-3,14-diaza-15-oxo-dispiro-(4,1,5,2)-pentadecan (2,5) | Jodethan (10) | dto. (4) | 5 | 55 | Heptan | 198 |
| 58[3]) | 137 | 2,2,4,4-Tetramethyl-7-oxa-3,20-diaza-21-oxo-dispiro-(5,1,11,2)-heneicosan (18,2) | Jodmethan (20) | dto. (30) | 8 | 45 | dto. | 179—181 |
| 59[3]) | 138 | dto. (10) | Bromethan | dto. (20) | 8 | 60 | Hexan | 159—160 |
| 60[3]) | 139 | dto. (18,5) | 2-Brompropan (20) | dto. (20) | 8 | 55 | Heptan | 141 |
| 61[3]) | 140 | dto. (10) | 1-Brombutan (20) | dto. (20) | 8 | 60 | Hexan | 124—126 |
| 62[3]) | 141 | dto. (18,2) | 2-Brombutan (20) | dto. (20) | 8 | 60 | dto. | 128—130 |
| 63[3]) | 143 | dto. (18,2) | 1-Bromhexan (20) | dto. (20) | 8 | 60 | dto. | 77—81 |
| 64[3]) | 142 | dto. (18,2) | 1-Brompentan (20) | dto. (20) | 8 | 60 | dto. | 122 |

Fortsetzung

| Bsp. Nr. | Verb. Nr. | Ausgangsmaterialien: Polyalkyl-1-oxa-diaza-spiro-decan; Menge (g) | Halogenverbindung Menge (g) | Base (ml) | Reaktionszeit (Stunden) | Temperatur (°C) | umkrist. aus | Fp. (°C) |
|---|---|---|---|---|---|---|---|---|
| 65[3]) | 144 | 2,2,4,4-Tetramethyl-7-oxa-3,20-diaza-21-oxo-dispiro-(5,1,11,2)-heneicosan (18,2) | 1-Bromoctan (20) | 50%ige NaOH (20) | 8 | 60 | Hexan | 80 |
| 66[3]) | 145 | dto. (18,2) | 1-Bromheptan (20) | dto. (20) | 8 | 60 | dto. | 88 |
| 67[3]) | 147 | dto. (18,2) | Benzylchlorid (20) | dto. (20) | 8 | 60 | Heptan | 155 |
| 68[3]) | 149 | dto. (18,2) | 1,5-Dibrompentan (5,7) | dto. (40) | 20 | 50 | dto. | 188 |
| 69[3]) | 150 | dto. (18,2) | 1,6-Dibromhexan (6,1) | dto. (20) | 20 | 50 | dto. | 198 |
| 70[3]) | 151 | dto. (36,4) | 1,10-Dibromdecan (15) | dto. (40) | 20 | 50 | dto. | 155 |
| 71 | 156 | 2,2,7,7-Hexamethyl-1-oxa-3,8-diaza-4-oxo-dispiro-(4,5)-decan (26,2) | 1,2-Bis-(chlor-naphthyl)-benzol (8,8) | dto. (30) | 30 | 60 | Petrolether | 102–104 |

[1]) ohne Lösungsmittel.
[2]) 20 ml Toluol als Lösungsmittel.
[3]) 150 ml Toluol als Lösungsmittel.

Beispiel 72

Dieses Beispiel zeigt die Flüchtigkeit der erfindungsgemäßen Stabilisatoren im Vergleich zu Produkten des Standes der Technik aus DE-OS 2 227 689.

Die Flüchtigkeiten wurden in einer Apparatur zur thermogravimetrischen Analyse bestimmt. Gleiche Menge (500 mg) der erfindungsgemäßen Stabilisatoren und die Vergleichssubstanzen wurden dazu in Stickstoffatmosphäre mit einer Aufheizgeschwindigkeit von 2 K/min bis auf 300° C aufgeheizt und der Substanzverlust in $mg/cm^2$ gemessen. Die Ergebnisse zeigt nachfolgende Tabelle:

| Stabilisator gemäß Beispiel | Gewichtsverlust in $mg/cm^2$ beim Erreichen von ...°C | | | |
|---|---|---|---|---|
| | 220 | 260 | 300 | 10 min bei 300 |
| 5 | 0,63 | 4,11 | 14,85 | 24,33 |
| 4 | 0,79 | 3,63 | 13,27 | 20,22 |
| 2 | 1,58 | 7,43 | 22,44 | 35,39 |
| 69 | 0,32 | 2,37 | 7,27 | 11,06 |
| 70 | 0,01 | 0,32 | 2,05 | 3,79 |
| Vergleich[1]) | 14,06 | 45,82 | 148,62 | 153,26 |

[1]) Verbindung nach Beispiel 58 der DE-OS 2 227 689.

Beispiel 73

Zum Nachweis der stabilisierenden Eigenschaften der neuen Verbindungen wurde wie folgt verfahren:

| | |
|---|---|
| 100 Gew.-Teile | Polypropylen mit einem Schmelzindex $i_5$ von ca. 6 g/10 min (bestimmt nach ASTM D 1238-62 T) und einer Dichte von 0,90 wurden mit |
| 0,1 Gew.-Teil | Pentaerythrityl-tetrakis-3-(3,5-di-tert.-butyl-4-hydroxyphenyl)-propionat, |
| 0,2 Gew.-Teilen | Calciumstearat und |
| 0,3 Gew.-Teilen | des zu prüfenden erfindungsgemäßen Stabilisators vermischt. |

Um eine möglichst gleichmäßige Verteilung auf dem Polymerkorn zu erreichen, wurden die Stabilisatoren in einem Lösemittel gelöst. Die Lösung wurde unter Rühren in das Polypropylenpulver eingetropft, wobei durch gleichzeitige Bestrahlung mit einer IR-Lampe der größte Teil des Lösemittels wieder abdampfte. Nach ca. 20 min wurde das Calciumstearat hinzugegeben und noch weitere 10 min gemischt. Lösemittelreste wurden durch Trocknen bei 50° C/120 min im Trockenschrank entfernt.

Das Polypropylen wurde auf einer Windsor-Spritzgußmaschine der Type SP 50 bei 240° C zu 60 x 60 x 1-mm-Platten verspritzt. Aus diesen Platten wurden Prüfkörper nach DIN 53 455, Form 3, verkleinert im Maßstab 1 : 3, ausgestanzt. Die als Vergleichsmuster benötigten Prüfkörper wurden analog, jedoch unter Fortlassen des zu testenden Stabilisators bzw. unter Zusatz der Vergleichsstabilisatoren, hergestellt.

Zur Bestimmung der Lichtstabilität wurden die Proben in einer Xenotest-1200-Apparatur der Firma Original Hanau Quarzlampen GmbH der Bestrahlung mit Wechsellicht unterworfen. Die Strahlungsintensität wurde durch UV-Filter (Spezialfilterglas d = 1,7 mm) moduliert. Die Lichtbeständigkeit wurde nach DIN 53 387 (17 min befeuchten, 3 min beregnen, Schwarztafeltemperatur 45° C, Luftfeuchtigkeit 70 bis 75%) geprüft. Gemessen wurde die Belichtungszeit in Stunden und die Reißdehnung bestimmt. Die Reißdehnung wurde auf einer Zugprüfmaschine der Firma Instron bei einer Abzugsgeschwindigkeit von 5 cm/min ermittelt.

| Stabilisator nach Beispiel | Belichtungszeit in Stunden | ermittelte Reißdehnung in % vom Ausgangswert |
|---|---|---|
| 5 | 1100 | >50 |
| 4 | 1100 | >50 |
| 2 | 1100 | >50 |
| 69 | 1100 | >50 |
| 70 | 1100 | >50 |
| Polypropylen | 260 | 1 |
| Vergleich (ohne Stabilisator) | 320 | 1 |
| Vergleich[1]) | 1100 | 2 |

[1]) Verbindung nach Beispiel 58 der DE-OS 2 227 689.

## Beispiel 74

Zu Polypropylen (Hostalen PPU VP 1770 F der Hoechst AG) vom Schmelzindex MFI 190/51,9 g/10 min s. DIN 53 535 werden 0,26 Gew.-Teile der unten angegebenen Stabilisatoren über einen Laborschnellmischer eingemischt. Das so stabilisierte Material wurde in einem Laborextruder unter den üblichen Verarbeitungsbedingungen aufgeschmolzen und über eine Spinnpumpe mit Mehrfachspinnkopf zu Monofilamenten (87 dtex) verarbeitet, welche anschließend im Verhältnis 1 : 2,5 nachverstreckt wurden. Je 24 dieser Filamente wurden zu Garn texturiert und dieses zu Prüfgeweben verarbeitet. Die Prüflinge wurden in einem Fadeometer der Lichtechtheitsprüfung unterzogen und nach der angegebenen Belichtungszeit dem Fingernageltest (leichtes Darüberreiben über das Gewebe mit dem Daumennagel) unterzogen. Der Grad des Abbaus wird durch Wertzahlen (0 = keine Schädigung, 1 bis 5 = zunehmende Zerstörbarkeit) ausgedrückt.

| Stabilisator gemäß Beispiel (Gew.-Teile) | Zerstörbarkeit des Gewebes nach ... Stdn. Belichtungszeit | | |
|---|---|---|---|
| | 40 | 80 | 160 |
| ohne Stab. (Vergleich) | 0 | 0 | 5 |
| 5 | 0 | 0 | 0 |
| 4 | 0 | 0 | 0 |
| 2 | 0 | 0 | 0 |
| 69 | 0 | 0 | 0 |
| 70 | 0 | 0 | 0 |
| Vergleich[1]) | 0 | 0 | 1 |

[1]) Stabilisator gemäß Beispiel 58 der DE-OS 2 227 689.

**Patentansprüche**

1. Polyalkyldiazaspirodecane der allgemeinen Formel (I)

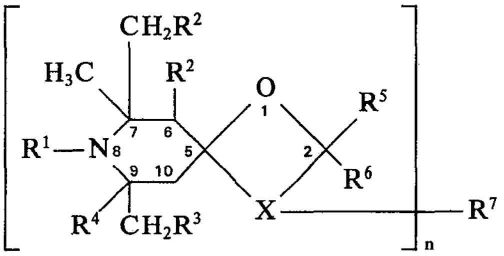

(I)

in welcher

X die Bedeutung einer Gruppe der Formel (II) oder (III) hat,

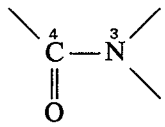
(II)

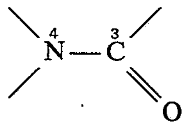
(III)

wobei die Indices 3 bzw. 4 die Ringpositionen im Diazaspirodecansystem angeben und die freie Valenz des Stickstoffatoms die Verknüpfung mit $R^7$ bewirkt,

$R^1$ Wasserstoff, Sauerstoff oder $C_1$- bis $C_{12}$-Alkyl ist,

$R^2$ und $R^3$ entweder gleich sind und Wasserstoff oder eine $C_1$- bis $C_5$-Alkylgruppe bedeuten, wobei dann

$R^4$ eine Methylgruppe ist, oder auch

$R^2$ Wasserstoff oder $C_1$- bis $C_5$-Alkyl ist und

$R^3$ und $R^4$ zusammen mit den Kohlenstoffatomen, an die sie gebunden sind, eine $C_5$- oder $C_6$-Cycloalkylgruppe oder eine Gruppe der Formel

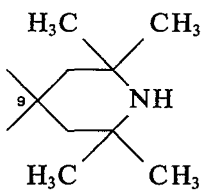

darstellen,

$R^5$ und $R^6$ gleich oder verschieden sind und für Wasserstoff, $C_1$- bis $C_{30}$-Alkyl, für eine unsubstituierte oder durch Chlor oder $C_1$- bis $C_4$-Alkyl substituierte Phenyl- oder Naphthylgruppe oder für eine unsubstituierte oder durch $C_1$- bis $C_4$-Alkyl substituierte $C_7$- bis $C_{12}$-Phenylalkylgruppe stehen, oder auch

$R^5$ und $R^6$ zusammen mit dem sie bindenden Kohlenstoffatom die Bedeutung einer unsubstituierten oder durch bis zu vier $C_1$- bis $C_4$-Alkylgruppen substituierten $C_5$- bis $C_{18}$-Cycloalkylgruppe oder einer Gruppe der Formel

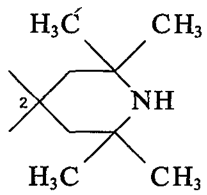

haben,

n 1 oder 2 bedeutet und

$R^7$ bei n = 1 eine Alkylgruppe mit 1 bis 30 Kohlenstoffatomen, eine Alkenylgruppe mit 2 bis 18 Kohlenstoffatomen, eine Phenylalkylgruppe mit 7 bis 18 Kohlenstoffatomen oder eine Cycloalkylgruppe mit 5 bis 12 Kohlenstoffatomen ist, wobei letztere Gruppen durch $C_1$- bis $C_4$-Alkylgruppen substituiert sein können, während

$R^7$ bei n = 2 eine Alkylengruppe mit 2 bis 30 Kohlenstoffatomen, eine Alkenylengruppe mit 2 bis 30 Kohlenstoffatomen oder eine Phenyldialkylengruppe mit 8 bis 18 Kohlenstoffatomen bedeutet,

sowie deren Salze mit nichtoxidierenden Mineralsäuren, aliphatischen Sulfon- oder Phosphonsäuren, aliphatischen Mono-, Di- und Polycarbonsäuren oder aromatischen Mono- und Polycarbonsäuren.

2. Verfahren zur Herstellung der Verbindungen nach Anspruch 1, dadurch gekennzeichnet, daß ein Polyalkyl-1-oxa-diaza-spirodecan der Formel (II)

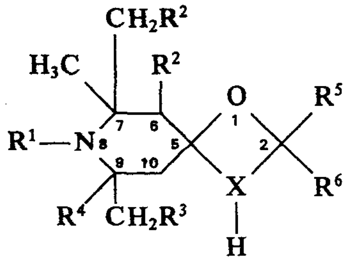

(II)

in der $R^1$, $R^2$, $R^3$, $R^4$, $R^5$, $R^6$ und X die im Anspruch 1 angegebenen Bedeutungen haben, mit einer Halogenverbindung der Formel (III)

$$R^7(Hal)_n \qquad (III)$$

in einem Zweiphasensystem, bestehend aus a) einem organischen Lösungsmittel und b) einem Alkalimetallhydroxid in fester Form oder wäßriger Lösung in Anwesenheit eines Phasentransferkatalysators bei 20 bis 120°C umsetzt, wobei die Edukte (II) und (III) im Falle von n = 1 im Molverhältnis 1 : 1 bis 1 : 10 und im Falle von n = 2 im Molverhältnis 2,5 : 1 bis 2 : 1 zum Einsatz kommen.

3. Verwendung der Verbindungen nach Anspruch 1 zum Stabilisieren von synthetischen Polymeren.

4. Verwendung nach Anspruch 3, dadurch gekennzeichnet, daß das Polymere ein Polyolefin, ein halogenhaltiges Polymeres, ein Polyacrylat oder Polymethacrylat oder ein Homo- oder Copolymeres des Styrols ist.

5. Gegen UV-Zersetzung stabilisierte synthetische Polymere, in denen 0,01 bis 5 Gewichtsteile, bezogen auf Polymeres, eines Stabilisators nach Anspruch 1 enthalten sind.

**Claims**

1. Polyalkyl-diaza-spirodecanes of the formula

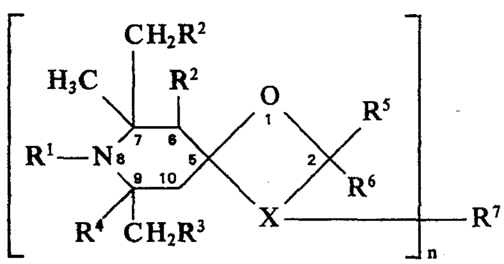

(I)

in which

X stands for a group of the formulae (II) or (III)

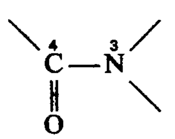

(II)

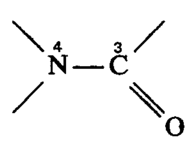

(III)

the indices 3 and 4 indicating the ring positions in the diaza-spirodecane system, and the free valency of the nitrogen atom producing the linkage with $R^7$;

$R^1$ is hydrogen, oxygen or $C_1-C_{12}$-alkyl,

$R^2$ and $R^3$ either are identical and represent hydrogen or a $C_1-C_4$-alkyl group, in which case

$R^4$ is a methyl group; or

$R^2$ is hydrogen or $C_1-C_5$-alkyl; and

$R^3$ and $R^4$, together with the carbon atoms to which they are linked, form a $C_5$- or $C_6$-cycloalkyl group or a group of the formula

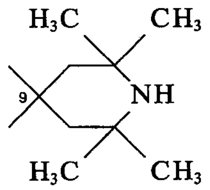

$R^5$ and $R^6$ are identical or different and represent hydrogen, $C_1-C_{30}$-alkyl, an unsubstituted phenyl or naphthyl group or a phenyl or naphthyl group substituted by chlorine or $C_1-C_4$-alkyl, or an unsubstituted $C_7-C_{12}$-phenylalkyl group or such a group substituted by $C_1-C_4$-alkyl, or

$R^5$ and $R^6$, together with the carbon atom linking them, form an unsubstituted $C_5-C_{18}$-cycloalkyl group or such a group substituted by up to four $C_1-C_4$-alkyl groups, or a group of the formula

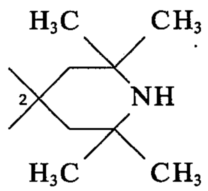

n is 1 or 2, and

$R^7$, in the case where n is 1, is an alkyl group having from 1 to 30 carbon atoms, an alkenyl group having from 2 to 18 carbon atoms, a phenylalkyl group having from 7 to 18 carbon atoms or a cycloalkyl group having from 5 to 12 carbon atoms; the latter groups possibly being substituted by $C_1-C_4$-alkyl groups; whilst

$R^7$ in the case where n is 2, is an alkylene group having from 2 to 30 carbon atoms, an alkenylene group having from 2 to 30 carbon atoms, or a phenyldialkylene group having from 8 to 18 carbon atoms;

and their salts with non-oxidizing mineral acids, aliphatic sulfonic or phosphonic acids, aliphatic mono-, di- or polycarboxylic acids, or aromatic mono- or polycarboxylic acids.

2. A process for the preparation of the compounds of the formula I, which comprises reacting a polyalkyl-1-oxa-diaza-spirodecane of the formula II

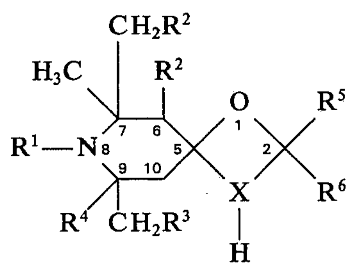

(II)

in which $R^1$ through $R^8$ and X are as defined in claim 1, with a halogen compound of the formula III

$$R^7(Hal)_n \qquad (III)$$

in a two-hase system, consisting substantially of a) an organic solvent and b) an alkali metal hydroxide, in solid form or aqueous solution, in the presence of a phase transfer catalyst, at 20—120° C; the educts (II) and (III) being used in a molar ratio of from 1 : 1 to 1 : 10 in the case where n is 1, and in a molar ratio of from 2.5 : 1 to 2 : 1 in the case were n is 2.

3. Use of the compounds as claimed in claim 1 for stabilizing synthetic polymers.

4. Use as claimed in claim 3 wherein the polymer is a polyolefin, a halogen-containing polymer, a polyacrylate or polymethacrylate, or a homo- or copolymer of styrene.

5. Synthetic polymers stabilized against decomposition by UV, which contain from 0.01 to 5 parts by weight, relative to the polymer, of a stabilizer as claimed in claim 1.

**Revendications**

1. Polyalkyl-diazaspirodécanes répondant à la formule générale (I):

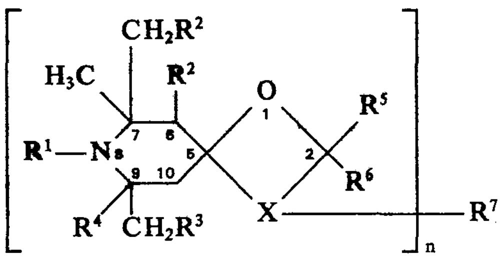

(I)

dans laquelle

X représente un radical répondant à l'une des formules (II) et (III):

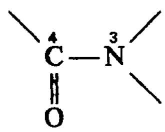

(II) et

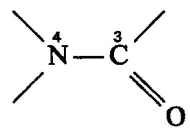

(III)

dans lesquelles les indices 3 et 4 indiquent les positions dans le système cyclique du diazaspirodécane et la valence libre de l'atome d'azote assure la liaison avec $R^7$,

$R^1$ représente l'hydrogène, l'oxygène ou un alkyle en $C_1-C_{12}$, ou bien

$R^2$ et $R^3$ sont identiques et représentent chacun l'hydrogène ou un alkyle en $C_1-C_5$, auquel cas

$R^4$ représente un radical méthyle, ou bien

$R^2$ représente l'hydrogène ou un alkyle en $C_1-C_5$ et

$R^3$ et $R^4$ forment ensemble et avec les atomes de carbone auxquels ils sont liés un radical cycloalkyle en $C_5$ ou $C_6$ ou un radical de formule:

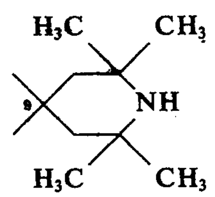

$R^5$ et $R^6$ sont identiques ou différents et représentent chacun l'hydrogène, un alkyle en $C_1-C_{30}$, un radical phényle ou naphthyle non substitué ou porteur d'un atome de chlore ou d'un alkyle en $C_1-C_4$, ou un radical phénylalkyle en $C_7-C_{12}$ non substitué ou porteur d'un alkyle en $C_1-C_4$, ou

$R^5$ et $R^6$ forment ensemble et avec l'atome de carbone qui les unit un radical cycloalkyle en $C_5-C_{18}$ non substitué et porteur d'au plus quatre radicaux alkyles en $C_1-C_4$, ou un radical de formule:

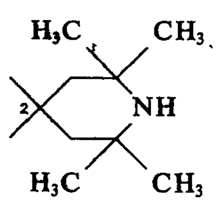

n est égal à 1 ou à 2 et

$R^7$ représente:

- lorsque n est égal à 1, un radical alkyle contenant de 1 à 30 atomes de carbone, un radical alcényle contenant de 2 à 18 atomes de carbone, un radical phénylalkyle contenant de 7 à 18 atomes de carbone ou un radical cycloalkyle contenant de 5 à 12 atomes de carbone, ces derniers groupes pouvant porter des alkyles en $C_1-C_4$, et
- lorsque n est égal à 2, un radical alkylène contenant de 2 à 30 atomes de carbone, un radical alcénylène contenant de 2 à 30 atomes de carbone ou un radical phényl-dialkylène contenant de 8 à 18 atomes de carbone,

ainsi que les sels qu'ils forment avec des acides minéraux non oxydants, des acides sulfoniques ou

phosphoniques aliphatiques, des acides mono-, di- ou polycarboxyliques aliphatiques ou des acides mono- ou polycarboxyliques aromatiques.

2. Procédé de préparation des composés selon la revendication 1, procédé caractérisé en ce qu'on fait réagir un polyalkyl oxa-1 diazaspirodécane répondant à la formule (II)

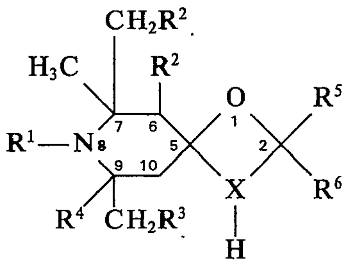

(II)

dans laquelle $R^1$, $R^2$, $R^3$, $R^4$, $R^5$, $R^6$ et X ont les significations données à la revendication 1, avec un composé halogéné répondant à la formule (III)

$$R^7(Hal)_n \quad \text{(III)}$$

dans un système à deux phases constitué a) d'un solvant organique et b) d'un hydroxyde de métal alcalin à l'état solide ou en solution aqueuse en présence d'un catalyseur de transfert de phases, à une température de 20 à 120° C, les corps de départ de formules (II) et (III) étant mis en jeu dans un rapport molaire de 1 : 1 à 1 : 10 dans le cas où n est égal à 1 et dans un rapport molaire de 2,5 : 1 à 2 : 1 dans le cas où n est égal à 2.

3. Application des composés selon la revendication 1 à la stabilisation de polymères synthétiques.

4. Application selon la revendication 3, caractérisée en ce que le polymère est une polyoléfine, un polymère contenant un halogène, un polyacrylate, un polyméthacrylate ou un homopolymère ou copolymère du styrène.

5. Polymères synthétiques stabilisés contre la destruction par les UV, polymères qui contiennent de 0,01 à 5 parties en poids, par rapport au polymère, d'un stabilisant selon la revendication 1.